# EUROPÄISCHE PATENTSCHRIFT

(11) **EP 0 597 282 B1**
(45) Veröffentlichungstag und Bekanntmachung des Hinweises auf die Patenterteilung: **07.06.1995**
(21) Anmeldenummer: 93116831.4
(22) Anmeldetag: 19.10.1993
(51) Int. Cl.: B65F 7/00

(54) **Sammelbehälter für organische Abfälle**
Collecting container for organic waste
Réceptacle collecteur de déchets organiques

(30) Priorität: 11.11.1992 DE 4238002; 21.11.1992 DE 4239191
(43) Veröffentlichungstag der Anmeldung: 18.05.1994
(73) Patentinhaber: DYNAMICS GESELLSCHAFT FÜR UMWELTSCHUTZ UND BIOLOGISCHE VERFAHRENSTECHNIK GmbH, D-48157 Münster (DE)
(72) Erfinder: Grodde, Günter, D-48157 Münster (DE)
(74) Vertreter: Habbel, Hans-Georg, Dipl.-Ing.

(56) Entgegenhaltungen:
- EP-A- 0 216 734
- EP-A- 0 388 517
- DE-U- 9 214 526
- US-A- 1 495 368

## Beschreibung

Die Erfindung bezieht sich auf einen Sammelbehälter für im Haushalt, Kleingewerbe oder dergleichen anfallende und der Kompostierung oder Vergärung zuzuführende organische Abfälle gemäß dem Oberbegriff des Hauptanspruches.

Grundsätzlich können die in Haushalten und bestimmten Gewerbebereichen anfallenden organischen Abfälle auf aerobem, thermophilem Weg durch Kompostierung oder auf anaerobem Weg durch fermentative, kalte Vergärung behandelt werden. Beide Verfahren haben in Abhängigkeit von der Zusammensetzung des zu behandelnden Abfalls Vor- oder Nachteile. Weisen die erfaßten Bioabfälle hohe Wassergehalte von etwa 80 % und mehr auf, wenn z. B. Gartenabfälle oder Abfälle aus Großmärkten fehlen, treten bei der Kompostierung Emissionsprobleme auf. In solchen Bereichen stellt die Vergärung ein besser geeignetes Behandlungsverfahren dar. Bioabfälle, die hohen Anteil an Gartenabfällen, insbesondere Strauch- und Baumschnitt aufweisen, können kaum vergoren werden, da Lignozellulose unter anaeroben Bedingungen nur begrenzt bzw. überhaupt nicht zu Biogas abgebaut werden kann.

Sammelbehälter für organische Abfälle werden in jüngster Zeit in zunehmendem Maße neben den sonst üblichen, den Haushalten zugeordneten Abfalltonnen eingesetzt, um damit die Menge des anfallenden Abfalls sinnvoll zu verringern, indem die in diesen sogenannten Biotonnen gesammelten Abfälle einer Kompostierung oder Vergärung zugeführt werden. Um diese organischen Abfälle zu sammeln, ist es auch im Haushalt erforderlich, neben dem üblichen Mülleimer einen "Biomülleimer" aufzustellen. Der Kompost oder das Biogas kann mit Gewinn wieder verkauft werden.

Ein Nachteil derartiger Biotonnen oder Biomülleimer ist aber, daß bereits in den Sammelbehältern eine Kompostierung dieser Abfallstoffe eintritt, die ein thermophiler aerober Vorgang ist, bei dem leicht abbaubare organische Substanzen unter intensiver Wärmeabgabe oxidiert werden. Zum Teil bilden sich aber auch in dem Sammelbehälter Zonen mit anaeroben Verhältnissen, in denen eine fermentative Vergärung stattfindet. Hier treten sehr unangenehm riechende gasförmige Zwischen- und Endprodukte auf. Diese anaeroben Vorgänge werden durch Luftausschluß durch zu hohe Feuchtigkeit bedingt. Hier tritt eine Hydrolyse ein, die zu Säure- und Gasbildungen führt. Beim anaeroben Abbau von Kohlehydraten entstehen vor allem Fettsäuren, Aldehyde, Ester und Alkohole, von denen einige sehr üble Geruchsempfindungen hervorrufen. Gleichzeitig werden mehr als 7 l Gas pro kg organischer Substanz gebildet. Die flüchtigen Substanzen bei der Vergärung oder Kompostierung zeichnen sich durch niedere Geruchsschwellenwerte und besondere Duftqualitäten aus, wobei wegen des niedrigen Geruchsschwellenwertes diese Verbindungen noch in hoher Verdünnung wahrnehmbar sind, d. h. sie stinken auch über weite Entfernungen.

Neben den lästigen Gerüchen entwickeln sich auch in Biotonnen Pilze, die zu Belästigungen und vor allem zur gesundheitlichen Gefährdung der Umwelt führen. Beim Öffnen der Sammelbehälter entweicht der durch die Kompostierung und Vergärung entstandene Überdruck, und gleichzeitig entsteht durch den Sog des Öffnens eine Verstärkung, wodurch die gesundheitsgefährdenden Pilzsporen hochgewirbelt und als Luftkeime leicht eingeatmet werden können.

Ein weiterer Nachteil bei den üblichen Sammelbehältern besteht darin, daß das aufsteigende, mit Feuchtigkeit gesättigte Gas an der Unterseite des Deckels kondensiert, wobei das Kondensat am Deckel zum Behälterrand und von dort an der Außenwand herabläuft, wodurch erhebliche Belästigungen in Form von Geruchsemmissionen und Verschmutzungen auftreten. Diese wiederum ziehen Ungeziefer wie z. B. Ratten und Mäuse an.

Aus der EP-A-216 734 ist ein Müllbehälter bekannt geworden, bei mit dem man die entstehenden und als lästig empfundenen Gerüche dadurch vermeiden will, daß in den Deckel ein zum Inneren des Behälters offener Aufnahmeraum ausgearbeitet ist, in den ein Desodorant eingefüllt wird, das also zum Inneren des Müllbehälters hin wirksam sein soll. Auch kann der Aufnahmeraum für das Desodorant auch nach außen hin offen sein, so daß die desodorierenden Dämpfe auch nach außen gelangen.

In der US-A-1 495 368 werden auch desinfizierende Mittel in den am Deckel vorgesehenen Aufnahmeraum eingefüllt.

Durch diese Anordnungen werden die Probleme einer Biotonne nicht vermieden, denn beim Öffnen des Müllbehälters treten sowohl die Pilze und deren Sporen wie auch die als lästig empfundenen Gase aus, und vor allen Dingen werden durch den Einsatz eines Desodorantes oder Desinfektionsmittels chemische Mittel eingetragen, die gerade bei Biotonnen nicht verwendet werden sollen. Diese Mittel enthalten Bakteriostatika, die den bakteriellen Abbau hemmen. Hier liegt genau die gegenteilige Wirkung eines Biofilters vor. Gewollt ist der bakterielle Abbau der Gase.

Der Erfindung liegt daher die Aufgabe zugrunde, in umweltfreundlicher und gleichzeitig kostengünstiger Weise die durch Biotonnen und Biomülleimer bedingten, die Umwelt belastenden Gerüche und Schadstoffe dadurch zu vermeiden, daß eine Abluftreinigung im Bereich der Biosammelbehälter erfolgt und sich kein Überdruck aufbauen kann sowie daß Schwitzwasser nicht nach außen dringt.

Diese der Erfindung zugrundeliegende Aufgabe wird durch die Lehre des kennzeichnenden Teils des Hauptanspruches gelöst.

Die biologische Reinigung von Abluft, bei der Schad- und Geruchsstoffe entfernt werden, erfolgt also durch Biofilter, wobei der Schadstoffabbau durch Mikroorganismen erfolgt, die auf einem festen Träger angesiedelt sind. Als Trägermaterial können Materialien mit großer Oberfläche eingesetzt werden, wie organische Materialien, oder anorganische, wie z. B. Lava oder Blähton, wobei diese normalerweise als Schüttstoffe vorliegenden Materialien durch Druck oder auch sonstige Hilfsmittel verfestigt sein können, so daß sie ein festes Filterbett bilden. Die luftverunreinigenden Stoffe aus der Abluft werden dort vorwiegend vom Wasser absorbiert. In einem simultan verlaufenden zweiten Schritt werden die sorbierten Substanzen auf der Oberfläche der Trägersubstanzen durch Mikroorganismen resorbiert und abgebaut. Die notwendigen Mikroorganismen und Enzyme können bereits am Trägermaterial immobilisiert sein oder werden nachträglich aufgeimpft.

Die Erfindung schlägt also mit anderen Worten ausgedrückt vor, daß beispielsweise in einer handelsüblichen Abfalltonne im Deckel eine Entlüftungsöffnung vorgesehen wird und daß am Deckel befestigt, vorzugsweise unterhalb dieser Entlüftungsöffnung, der eigentliche Biofilter eingesetzt wird. Der Biofilter besteht dabei vorzugsweise aus einem Filterbett und einem dieses Filterbett im Randbereich umgebenden Feuchtmaterial, das dafür Sorge trägt, daß stets genügend Feuchtigkeit zur Aktivierung des Filterbettes vorhanden ist.

Bei einer ersten Ausführungsform der Erfindung wird so vorgegangen, daß unterhalb der Entlüftungsöffnung im Deckel des Sammelbehälters eine Abdeckplatte vorgesehen wird und daß im Randbereich des eigentlichen Filterbetts das Feuchtmaterial angeordnet wird. Wird nunmehr durch die Entlüftungsöffnung Wasser in den Sammelbehälter eingeführt, tränkt dieses Wasser das Feuchtmaterial, und dieses Feuchtmaterial gibt anschließend das Wasser langsam an das Filterbett ab. Es kann aber auch so vorgegangen werden, daß das Filterbett von einer luft- und wasserdurchlässigen Schicht umschlossen wird, an der außen das Feuchtmaterial anliegt, so daß beim Befeuchten sowohl das Filterbett wie auch das Feuchtmaterial mit Wasser angefeuchtet wird.

Der eigentliche Biofilter ist leicht auswechselbar im Deckel der Abfalltonne oder des Sammelbehälters angeordnet und kann so bei Erschöpfung schnell ausgewechselt werden. Die Entsorgung des eigentlichen Biofilters erfolgt ebenfalls im Wege der Kompostierung.

Da der Deckel des Sammelbehälters durch das Gewicht des Biofilters und der zugehörigen Bauteile erhöht wird, preßt sich der Deckel abdichtend auf die Oberkante des Unterteils des Sammelbehälters, und wenn in diesem Bereich zusätzlich eine komprimierbare Dichtung angeordnet wird, erfolgt ein nahezu falschluftdichtes Abschließen des Sammelbehälters, so daß die entstehenden Abgase ausschließlich durch den Biofilter und die Entlüftungsöffnung austreten müssen.

Die Anbringung des Biofilters am Deckel hat den Vorteil, daß durch die Deckelbewegung eine Durchlüftung und Durchfeuchtung des Filters erreicht wird, d. h. diese Durchlüftung und Durchfeuchtung wird Zwangsläufig immer dann erreicht, wenn der Abfalleimer oder die Biotonne benutzt werden.

Durch die Entlüftungsöffnung im oberen Teil können die Abgase, nachdem sie ihre Feuchtigkeit an den Biofilter abgegeben haben und von Schadstoffen gereinigt worden sind, ungehindert nach außen gelangen. Somit kann man hier von einem "offenen System" sprechen.

Im Gegensatz zu den "geschlossenen Systemen" der üblichen Sammelbehälter kann sich somit kein Überdruck aufbauen. Hierdurch wird das gesundheitsgefährdende Aufwirbeln der Pilzsporen vermieden. Ebenso wird das bei den geschlossenen Systemen typische Abtropfen des Kondensats an den Außenwandungen der Sammelbehälter vermieden, und zwar einerseits durch die Aufnahme der Feuchtigkeit durch den Biofilter mit seiner Abdeckplatte und andererseits - falls die Filter- und Feuchtmaterialien gesättigt sind - durch Abtropfen nach innen aufgrund der Ausbildung (Tropfkante) der Stützvorrichtung oder der Wände des Biofilters.

Bei den "offenen Systemen" der Sammelbehälter, die zwar Belüftungsöffnungen aber keine Abdichtung haben, damit der Gasfluß nur von außen nach innen erfolgen kann, hat die Praxis gezeigt, daß starke Geruchsemissionen durch diese Öffnungen erfolgen. Ebenso tritt Feuchtigkeit mit den zwangsläufigen Verschmutzungen aus. Diese ziehen Ungeziefer wie z. B. Ratten und Mäuse an.

Als weiterer Nachteil wurde erkannt, daß Insekten und Kleintiere durch diese nicht abgedichteten Belüftungsöffnungen in das Innere der Sammelbehälter gelangen. Hier finden sie ideale Lebensbedingungen vor, da trotz regelmäßiger Entleerung erhebliche Reste von Abfallstoffen am Boden und an den Innenwänden haften.

Die bei diesen Systemen angestrebte Austrocknung der anaeroben Zonen erfolgt nur unzureichend, da keine zwangsläufige Entlüftung nach oben erfolgt. Dies ist nur möglich, wenn die Entlüftungsöffnung z. B. durch eine Membran so abgedichtet wird, daß ein Gasfluß nur von außen nach innen zugelassen wird.

Als Schutz gegen überschüssige Feuchtigkeit von außen kann die Belüftungsöffnung so abgedeckt werden, daß sie ganz oder teilweise z. B. durch einen Drehschieber manuell verschlossen wird.

Ein Ausführungsbeispiel der Erfindung wird nachfolgend anhand der Zeichnungen erläutert. Die Zeichnungen zeigen dabei in
- Fig. 1: schaubildlich einen Sammelbehälter mit eingebautem Biofilter, in
- Fig. 2: in einer herausgezogenen Darstellungsweise den Biofilter gemäß Fig. 1, in
- Fig. 3: in einer Schnittdarstellung eine abgeänderte Ausführungsform eines Biofilters und in
- Fig. 4: einen Ausschnitt aus einem Sammelbehälterdeckel, bei dem sichergestellt ist, daß zwar eine Belüftung im Deckel erfolgen kann, aber Regenwasser nicht eindringen kann.

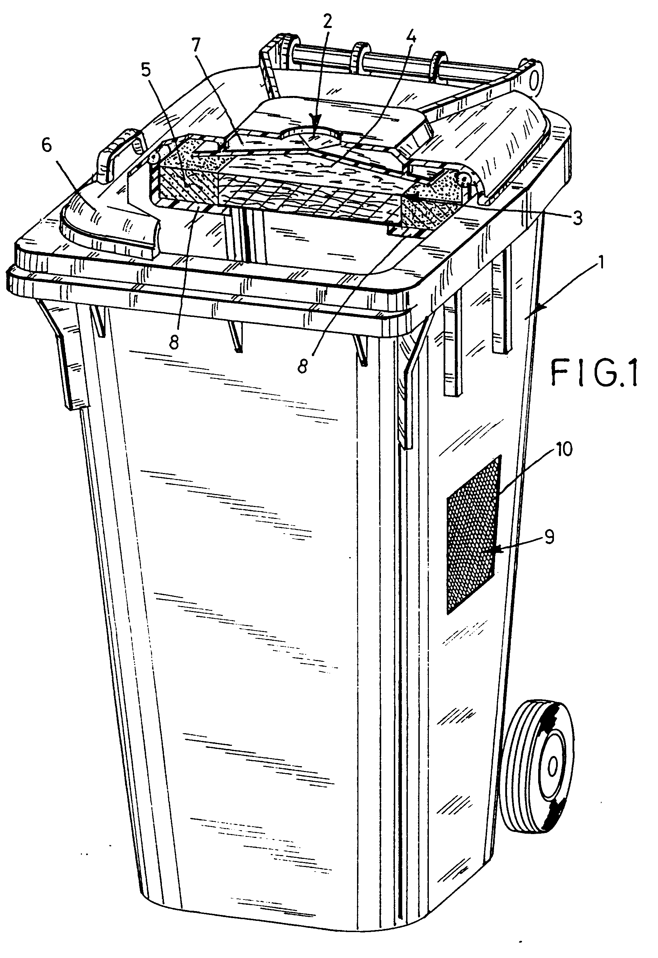

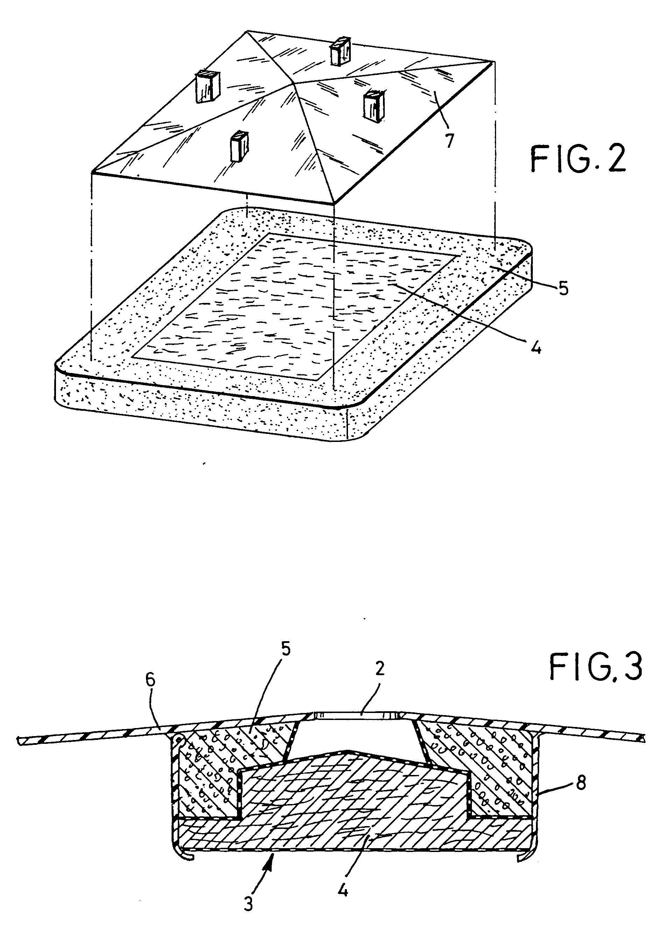

In den Zeichnungen ist mit 1 ein Sammelbehälter bezeichnet, der nach oben durch einen Deckel 6 verschlossen wird, wobei in diesem Deckel 6 eine Entlüftungsöffnung 2 vorgesehen wird. Unterhalb der Entlüftungsöffnung 2 ist bei der Ausführungsform gemäß Fig. 1 und Fig. 2 eine Abdeckplatte 7 angeordnet, die beispielsweise fest an der Unterseite des Deckels 6 vorgesehen sein kann. Unterhalb der Abdeckplatte 7 ist der eigentliche Biofilter 3 vorgesehen, das aus einem Filterbett 4 und einem dieses Filterbett 4 umgebenden Feuchtmaterial 5 besteht. Filterbett 4 und Feuchtmaterial 5 sind bei der in Fig. 1 und 2 dargestellten Ausführungsform plattenförmig ausgebildet, und der äußere Randbereich der Abdeckplatte 7 reicht bis etwa über die Mitte des Feuchtmaterials 5, so daß beim Einfüllen von Wasser durch die Entlüftungsöffnung 2 oder bei Eindringen von Regen durch die Entlüftungsöffnung 2 dieses Wasser in das Feuchtmaterial 5 geleitet wird und nicht das Filterbett 4 direkt durchströmt.

Das Feuchtmaterial 5 sorgt dafür, daß die hier gespeicherte Feuchtigkeit langsam an das Filterbett 4 abgegeben wird, so daß die im Filterbett 4 angesiedelten Mikroorganismen ausreichende Lebensbedingungen finden. Die im Feuchtmaterial 5 notwendige Feuchtigkeit kann auch aus den Abfällen mit den Abgasen in das Feuchtmaterial 5 eingetragen werden.

Der Biofilter 3, d. h. das Feuchtmaterial 5 und das Filterbett 4, wird auf entsprechenden Stützvorrichtungen 8 an der Unterseite des Deckels 6 des Sammelbehälters 1 gehalten, wobei diese Stützvorrichtungen 8 abgeklappt oder abgenommen werden können, so daß ein Auswechseln des Biofilters 3 möglich ist. Hierbei kann so vorgegangen werden, daß ausschließlich das Filterbett 4 ausgewechselt wird, es kann aber auch vorgesehen sein, daß das Filterbett 4 zusammen mit dem Feuchtmaterial 5 ausgewechselt wird.

Bei der in Fig. 3 dargestellten Ausführungsform ist unterhalb des Deckels 6 und unterhalb der Entlüftungsöffnung 2 der Biofilter 3 angeordnet, der auch hier aus dem Filterbett 4 und dem Feuchtmaterial 5 besteht. In dem dort in Fig. 3 dargestellten Ausführungsbeispiel ist keine Abdeckplatte 7 vorgesehen, sondern das Filterbett 4 und das Feuchtmaterial 5 werden von einem luft- und feuchtigkeitsdurchlässigen Werkstoff umschlossen, der ein direktes Durchfließen von Wasser durch das Filterbett verhindert aber gleichzeitig sicherstellt, daß ein Nachfeuchten des Filterbettes durch Feuchtigkeit aus dem Feuchtmaterial 5 möglich ist.

Die bei der beginnenden Zersetzung der organischen Abfälle innerhalb des Sammelbehälters sich bildenden Abgase steigen zwangsläufig nach oben, durchdringen das Filterbett 4, befeuchten dieses gegebenenfalls und verlassen den Sammelbehälter 1 durch die Entlüftungsöffnung 2 und sind nach dem Durchdringen des Biofilters 3 von luftverunreinigenden Stoffen befreit. Zur besseren Belüftung des Sammelbehälters 1 kann eine Belüftungsöffnung 9 vorgesehen sein, die durch eine Membrane 10 abgedichtet ist, die nur einen Gasfluß von außen nach innen zuläßt. Hierdurch wird die Bildung anaerober Zonen vermindert.

Bekannt sind Sammelbehälter für Bioabfälle mit Belüftungsöffnungen, die einen Gasfluß von außen nach innen aber auch umgekehrt zulassen. Die Praxis hat gezeigt, daß derartige Öfnungen nicht den gewünschten Effekt erzielen, nämlich die anaeroben Zonen zu durchlüften, da der Gasfluß nicht zwangsläufig durch den Abfall von unten nach oben erfolgt.

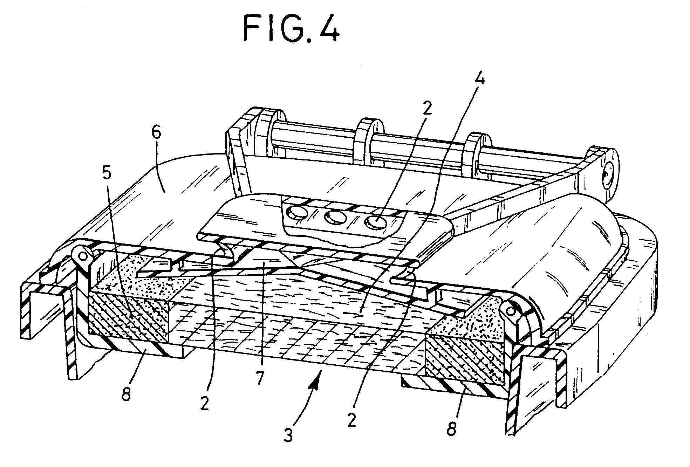

Fig. 4 zeigt, daß die Ausbildung des Deckels 6 so erfolgen kann, daß die Belüftungsöffnung 2 so angeordnet sind, daß einerseits das Eindringen von Regen nicht möglich ist, andererseits aber gleichzeitig durch die kaminartige Wirkung das Innere des Behälters intensiver entlüftet bzw. durchlüftet wird.

In der Zeichnung nicht dargestellt ist eine weitere Möglichkeit der Gestaltung der Öffnung 2 so, daß die beispielsweise in Fig. 3 dargestellte Öffnung 2 mit einem Schieberverschluß ausgerüstet ist, der ein Öffnen oder Verschließen ermöglicht, so daß bei langanhaltenden Regenfällen die Öffnung 2 verschlossen werden kann, andererseits aber bei langanhaltender Trockenheit durch die Öffnung 2 auch Wasser zum Feuchtmaterial 5 zugegeben werden kann.

## Patentansprüche

1. Sammelbehälter für der Kompostierung oder Vergärung zuzuführende organische Abfälle aus Haushalt, Kleingewerbe od. dgl. mit einer im Deckel (6) des Sammelbehälters (1) angeordneten Entlüftungsöffnung (2) und einem innerhalb des Sammelbehälters (1) vor der Entlüftungsöffnung auswechselbar angeordneten Filter, dadurch gekennzeichnet, daß der Filter als Biofilter (3) ausgebildet ist, in welchem gas- und aerosolförmige luftverunreinigende Stoffe aerob durch Mikroorganismen abgebaut werden.

2. Sammelbehälter nach Anspruch 1, gekennzeichnet durch ein das Filterbett (4) des Biofilters (3) umgebendes Feuchtmaterial (5), das in der Lage ist, Feuchtigkeit zu speichern und abzugeben.

3. Sammelbehälter nach Anspruch 1 oder 2, dadurch gekennzeichnet, daß das Filterbett (4) und das Feuchtmaterial (5) plattenförmig ausgebildet sind und oberhalb des Filterbettes (4) und unterhalb der Entlüftungsöffnung (2) eine wasserabweisende Ablenkplatte (7) vorgesehen ist, die das durch die Entlüftungsöffnung (2) eindringende Wasser dem Feuchtmaterial (5) zuführt und Kondensat nach unten abführt.

4. Sammelbehälter nach einem der vorhergehenden Ansprüche, gekennzeichnet durch eine zwischen dem oberen Rand des Sammelbehälters (1) und der mit diesem Rand zusammenwirkenden Unterseite des Deckels (6) angeordnete Dichtung, die vom Deckel (6) dichtend auf den oberen Rand des Sammelbehälters (1) gepreßt wird.

5. Sammelbehälter nach einem der vorhergehenden Ansprüche, dadurch gekennzeichnet, daß die Entlüftungsöffnung (2) gegen Eindringen von Regen gesichert ist.

6. Sammelbehälter nach einem der vorhergehenden Ansprüche, dadurch gekennzeichnet, daß die Stützvorrichtung (8) für das Biofilter (3) mit Tropfkanten derart ausgerüstet ist, daß das Kondenswasser in das Innere des Behälters abgeleitet wird.

7. Sammelbehälter nach einem der vorhergehenden Ansprüche, gekennzeichnet durch eine oder mehrere, in den Seitenwänden des Sammelbehälters (1) angeordnete Belüftungsöffnung (9), die durch eine Membrane (10) verschlossen sind, die nur einen Gasfluß von außen nach innen zuläßt.

## Claims

1. A collecting vessel for organic household or small-scale industrial refuse or the like for composting or fermentation, comprising a ventilation opening (1) arranged in the lid (6) of the collecting vessel (1) and a filter arranged replaceably inside the collecting vessel (1) in front of the ventilation opening, characterized in that the filter is constructed as a biofilter (3), in which gaseous and aerosol-form air-polluting substances are aerobically degraded by microorganisms.

2. A collecting vessel according to claim 1, characterized by a moisture material (5) surrounding the filter bed (4) of the biofilter (3), which moisture material (5) is in a position to store and release moisture.

3. A collecting vessel according to claim 1 or claim 2, characterized in that the filter bed (4) and the moisture material (5) are of sheet-form construction and in that a water-repellent baffle plate (7) is provided above the filter bed (4) and below the ventilation opening (2), which baffle plate (7) conveys the water penetrating through the ventilation opening (2) to the moisture material (5) and carries condensate away downwards.

4. A collecting vessel according to any one of the preceding claims, characterized by a seal arranged between the upper edge of the collecting vessel (1) and the underside, interacting with this edge, of the lid (6), which seal is pressed by the lid (6) sealingly onto the upper edge of the collecting vessel (1).

5. A collecting vessel according to any one of the preceding claims, characterized in that the ventilation opening (2) is secured against rain penetration.

6. A collecting vessel according to any one of the preceding claims, characterized in that the supporting device (8) for the biofilter (3) is provided with drip edges in such a way that the condensation water is conveyed away into the inside of the vessel.

7. A collecting vessel according to any one of the preceding claims, characterized by one or more ventilation openings (9) arranged in the side walls of the collecting vessel (1), which ventilation openings (9) are closed by a membrane (10) which only permits a gas flow from the outside inwards.

## Revendications

1. Réceptacle collecteur pour le compostage ou la fermentation de déchets organiques à évacuer provenant de ménages, de petites entreprises ou similaires, avec une ouverture d'aération (2) disposée dans le couvercle (6) du réceptacle collecteur (1) et un filtre disposé de façon interchangeable à l'intérieur du réceptacle collecteur (1) devant l'ouverture d'aération, caractérisé en ce que le filtre est conçu comme un biofiltre (3), dans lequel les substances gazeuses et les aérosols polluant l'air sont décomposés par voie aérobie par des micro-organismes.

2. Réceptacle collecteur selon la revendication 1, caractérisé en ce qu'il comporte un matériau humide (5) entourant le lit filtrant (4) du biofiltre (3) et capable d'emmagasiner et de libérer de l'humidité.

3. Réceptacle collecteur selon la revendication 1 ou 2, caractérisé en ce que le lit filtrant (4) et le matériau humide (5) sont en forme de plaque et qu'il est prévu au-dessus du lit filtrant (4) et en-dessous de l'ouverture d'aération (2) une plaque de déviation (7) détournant l'eau, qui amène l'eau pénétrant par l'ouverture d'aération (2) au matériau humide (5) et évacue le condensat vers le bas.

4. Réceptacle collecteur selon l'une ou l'ensemble des revendications précédentes, caractérisé en ce qu'il comporte un joint disposé entre le bord supérieur du réceptacle collecteur (1) et la face inférieure du couvercle (6) coopérant avec ce bord, qui est appuyé de manière étanche par le couvercle (6) sur le bord supérieur du réceptacle collecteur (1).

5. Réceptacle collecteur selon l'une ou l'ensemble des revendications précédentes, caractérisé en ce que l'ouverture d'aération (2) est protégée contre la pénétration d'eau de pluie.

6. Réceptacle collecteur selon l'une ou l'ensemble des revendications précédentes, caractérisé en ce que le dispositif d'appui (8) pour le biofiltre (3) est équipé de bords d'écoulement, de telle sorte que l'eau de condensation soit évacuée vers l'intérieur du réceptacle.

7. Réceptacle collecteur selon l'une ou l'ensemble des revendications précédentes, caractérisé en ce qu'il comporte une ou plusieurs ouvertures d'aération (9) disposées dans les parois latérales du réceptacle collecteur (1), qui sont fermées par une membrane (10) qui ne permet la circulation des gaz que de l'extérieur vers l'intérieur.
